# EUROPEAN PATENT SPECIFICATION

(11) **EP 3 075 317 B1**
(45) Date of publication and mention of the grant of the patent: **20.09.2017**
(21) Application number: 15162201.6
(22) Date of filing: 01.04.2015
(51) Int. Cl.: A61B 6/00, A61B 6/04

(54) **X-RAY SYSTEM AND METHOD FOR OPERATING AN X-RAY SYSTEM**
RÖNTGENSYSTEM UND VERFAHREN ZUM BETRIEB EINES RÖNTGENSYSTEMS
SYSTÈME À RAYONS X ET PROCÉDÉ POUR FAIRE FONCTIONNER UN SYSTÈME À RAYONS X

(43) Date of publication of application: 05.10.2016
(73) Proprietor: Agfa HealthCare N.V., 2640 Mortsel (BE)
(72) Inventor: Zemánek, Vladimír, 85579 Neubiberg (DE); Gerstlauer, Bernd, 81541 München (DE); Zehetmaier, Thomas, 85646 Neufarn (DE); Von Stein, Florian, 82194 Gröbenzell (DE); Von Stein, Ruben, 82194 Gröbenzell (DE)

(56) References cited:
- WO-A1-2010/128417
- DE-A1- 3 236 116
- JP-A- H09 238 931
- JP-A- 2011 125 580
- US-A1- 2014 163 734
- US-B1- 8 975 854

## Description

The present invention relates to an X-ray system and a method for operating an X-ray system according to the independent claims.

X-ray systems for medical imaging applications typically comprise an X-ray source, an X-ray detector and an X-ray table or wall stand, on which the X-ray detector is provided.

EP 2 427 110 B1 relates to a motor assisted manually controlled movement assembly for moving elements and devices of X-ray systems or patient support systems, comprising a motor which is adapted to detect a manual indication of a desired movement of a second structural element relative to a first structural element by means of determining an applied torque to the motor by a resulting motor current.

It is an object of the present invention to provide an improved X-ray system and an according method for operating an X-ray system.

The object is achieved by the X-ray system and the method for operating an X-ray system according to the independent claims.

The X-ray system according to one aspect of the invention comprises: a first structure, on which an X-ray source and/or an X-ray detector is provided; a second structure; at least one stepper motor, which is configured to move the first structure relative to the second structure, the stepper motor comprising a rotor shaft which is coupled with the first structure and/or the second structure such that when a user exerts an external force on the first structure an angular position of the rotor shaft is changed in accordance with the exerted external force; at least one incremental rotary encoder, which is coupled with the rotor shaft of the stepper motor and configured to generate an encoder signal in accordance with the change of the angular position of the rotor shaft; and a control unit, which is configured to determine, based on the encoder signal, a position deviation signal characterizing a deviation of the angular position of the rotor shaft from a reference angular position of the rotor shaft and to control the stepper motor to rotate the rotor shaft dependent on the position deviation signal such that the stepper motor supports a movement of the first structure relative to the second structure in accordance with the exerted external force.

The according inventive method for operating an X-ray system, which comprises a first structure, on which an X-ray source and/or an X-ray detector is provided, a second structure and at least one stepper motor comprising a rotor shaft that is coupled with the first structure and/or the second structure, comprises the following steps: changing an angular position of the rotor shaft in accordance with an external force, which is exerted on the first structure by a user; generating an encoder signal by means of an incremental rotary encoder, which is coupled with the rotor shaft of the stepper motor, in accordance with the change of the angular position of the rotor shaft; determining, based on the encoder signal, a position deviation signal characterizing a deviation of the angular position of the rotor shaft from a reference angular position of the rotor shaft; and controlling the stepper motor to rotate the rotor shaft dependent on the position deviation signal such that the stepper motor supports a movement of the first structure relative to the second structure in accordance with the exerted external force.

For example, the first structure can comprise a housing which is configured to accommodate the X-ray source and/or a telescope arm on which the housing is provided and/or a carriage on which the telescope arm is provided. Alternatively or additionally, the first structure can comprise a housing or holder, e.g. a so-called bucky, which is configured to accommodate the X-ray detector.

For example, the second structure can comprise one or more rails or tracks on which the carriage is moveably mounted. Alternatively or additionally, the second structure can comprise an X-ray table or X-ray stand, in particular a wall stand, on which the holder or housing accommodating the X-ray detector is moveably mounted. Preferably, the second structure, e.g. the rails, tracks, X-ray table or X-ray stand, may be fixed on the ceiling and/or on the bottom and/or on a wall.

Regarding the first and second structure a number of further combinations of relatively moveable components of X-ray systems are possible. For example, the housing accommodating the X-ray source (first structure) can be rotated around a horizontal axis relative to a so-called gooseneck (second structure), on which the housing is rotatably mounted. According to another example, the gooseneck (first structure) can be rotated around a vertical axis relative to a telescope (second structure), on which the gooseneck is rotatably mounted. According to yet another example, a carriage (first structure), on which a holder or housing accommodating the X-ray detector is provided, can be moved along a vertical axis relative to a vertical pillar (second structure) of a wall stand. Alternatively or additionally, the X-ray detector holder or housing (first structure) can be rotated around a horizontal axis relative to the carriage (second structure), on which the holder or housing is rotatably mounted.

Within the meaning of the invention, the term "stepper motor" preferably relates to an electric motor that divides a full rotation of the rotor or motor shaft into a plurality of equal steps so that the angular position of the motor shaft can be commanded to move and hold at one of these steps. In distinction to usual DC motors rotating continuously when voltage is applied to their terminals, a stepper motor converts a train of input pulses, e.g. square wave pulses (for full-step operation) or quasi sine wave signal (for micro stepping operation), into a precisely defined increment in the shaft position. Each pulse moves the shaft through a fixed angle. Preferably, the stepper motor has a plurality of toothed electromagnets arranged around a central gear-shaped piece of iron. The electromagnets are energized by an external control circuit, such as a stepper driver controlled by a microcontroller. To make the motor shaft turn, first, one electromagnet is given power, which magnetically attracts the gear's teeth. When the gear's teeth are aligned to the first electromagnet, they are slightly offset from the next electromagnet. This means that when the next electromagnet is turned on and the first is turned off, which is also referred to as commutation, the gear rotates slightly to align with the next one. From there the process is repeated. Each of those rotations is called a "step", with an integer number of steps making a full rotation. In that way, the motor can be turned by a precise angle. If the motor phases are energized but not commutated, the motor shaft holds its position with a holding torque and works like a static brake.

Within the meaning of the invention, the term "incremental rotary encoder" preferably relates an electro-mechanical device that converts the relative angular position or motion of a shaft or axle to a digital code. In distinction to a so-called absolute rotary encoder which provides information about the absolute position of the shaft and/or an assembly driven by the shaft, an incremental rotary encoder provides cyclical outputs only when the encoder shaft is rotated, i.e. when the angular position of the shaft changes. Accordingly, the signal output of an incremental rotary encoder provides information about a motion of the shaft.

The invention is based on the approach to provide at least one stepper motor by which a movement of the first structure and the second structure relative to each other is driven, preferably via an appropriate driving means, like at least one driving wheel and/or threaded spindle and/or driving gear and/or cable control. The rotor shaft of the stepper motor is coupled with the first and/or second structure, preferably via driving means, such that when a user exerts a manual force on the first structure, whereupon the first structure is displaced relative to the second structure by a, preferably infinitesimally, small distance, an according angular displacement of the rotor shaft from a reference angular position of the motor shaft, e.g. from a stop position or an initial position of the motor shaft, is caused. The deviation of the current angular position of the rotor shaft from the reference angular position is determined based on an encoder signal of an incremental rotary encoder which is coupled with the rotor shaft. The determined deviation of the current angular position of the rotor shaft from the reference angular position is a measure of the manual force applied to the first structure and serves as a basis for controlling the stepper motor to rotate the rotor shaft such that a movement of the first structure relative to the second structure is supported in accordance with the magnitude and/or direction of the exerted manual force. For example, if the user exerts a manual force on the first structure in an x direction, an according deviation of the current angular position of the rotor shaft from the reference angular position is detected. Then the stepper motor is energized to rotate the rotor shaft in the direction of the detected deviation of the current angular position, whereby the first structure is driven to move relative to the second structure in the x direction. In this way, a movement of the first structure relative to the second structure in x direction is supported by the stepper motor drive. Same applies accordingly for cases in which the user exerts a manual force in a y and/or z direction and/or a manual torque around a rotatory axis or a manual force or torque comprising force or torque components, respectively, in or around the x and/or y and/or z direction. It is preferred that, only if a certain threshold of angular position deviation is reached, the stepper motor is controlled in such a way that it generates a constant supporting torque.

By providing an incremental rotary encoder for detecting the current angular position of the rotor shaft and using the according encoder signals for determining a deviation of the current angular position of the rotor shaft from the reference angular position a manual force applied on the first structure indicating a desired movement of the first structure can be determined with substantially higher resolution and accuracy and a simpler electronic circuit compared to prior art solutions. In combination with a stepper motor drive a particularly precise positioning and movement of the first structure relative to the second structure in accordance with the exerted manual force is enabled.

All in all, the invention provides an improved X-ray system and method for operating an X-ray system compared to the ones known in the prior art.

According to a preferred embodiment, the control unit is configured to determine whether the position deviation signal exceeds a predefined value and, if the position deviation signal exceeds the predefined value, to control the stepper motor to rotate the rotor shaft dependent on the position deviation signal such that the stepper motor supports a movement of the first structure relative to the second structure in accordance with the exerted external force. By defining a threshold value, the system can be easily configured to support a movement of the first structure relative to the second structure only in cases in which the manual force exerted on the first structure exceeds a certain force. In this way, an unintentional start of a movement of the first structure can be avoided.

According to another preferred embodiment, the control unit is configured to control the stepper motor to rotate the rotor shaft dependent on the sign of the position deviation signal such that the stepper motor supports a movement of the first structure relative to the second structure in accordance with the exerted external force. For example, the rotor shaft is rotated in a forward direction, in cases where the position deviation signal is positive, and the rotor shaft is rotated in a backward direction, in cases where the position deviation signal is negative. In this way, a particularly intuitive control and/or support of the movement of the first structure relative to the second structure by means of applying a manual external force on the first structure is enabled.

According to yet another preferred embodiment, the control unit is configured to determine, based on the encoder signal generated during the support of the movement of the first structure relative to the second structure, an angular velocity of the rotor shaft and whether the angular velocity of the rotor shaft exceeds or falls below a predefined value and, if the angular velocity of the rotor shaft exceeds or falls below the predefined value, to control the stepper motor to stop supporting the movement of the first structure relative to the second structure.

Alternatively or additionally, it is preferred that the control unit is configured to determine, based on the encoder signal generated during the support of the movement of the first structure relative to the second structure, an angular velocity of the rotor shaft and whether the angular velocity of the rotor shaft exhibits a decrease that exceeds a predefined value and, if the decrease of the velocity of the rotor shaft exceeds the predefined value, to control the stepper motor to stop supporting the movement of the first structure relative to the second structure. The aforementioned preferred embodiments, alone or in combination, also allow for controlling the movement of the first structure relative to the second structure easily and intuitively.

It is, moreover, preferred that the control unit is configured to generate a reference position signal corresponding to an initial reference angular position of the rotor shaft. Preferably, the initial reference angular position of the rotor shaft is a time-invariant and/or fixed angular position. The initial reference angular position of the rotor shaft preferably corresponds to a current rest position of the first structure. That is, the reference position signal preferably corresponds to a current rest position or initial position of the first structure. When a manual force is exerted on the first structure resting in the rest position or initial position, the incremental rotary encoder generates an according encoder signal from which, in consideration of the reference position signal, a position deviation signal is determined. Based on the position deviation signal the stepper motor is controlled to rotate the rotor shaft such that a movement of the first structure relative to the second structure in accordance with the exerted force is supported.

According to another preferred embodiment, the control unit is configured to generate a reference position signal corresponding to a time-varying reference angular position of the rotor shaft. Preferably, the time-varying reference angular position of the rotor shaft corresponds to a varying position of a moving first structure relative to the second structure, so that the reference position signal corresponds to a time-varying position of the moving first structure. When a manual force is exerted on the moving first structure, the incremental rotary encoder generates an according encoder signal from which a position deviation signal is determined in consideration of the reference position signal. Based on the position deviation signal, the stepper motor is controlled to rotate the rotor shaft such that a movement of the first structure relative to the second structure in accordance with the exerted force is supported. In distinction to the previous embodiment, where a deviation of the angular position of the rotor shaft from a time-invariant or fixed reference angular position is determined, a deviation of the angular position of the rotor shaft from a time-varying reference angular position is determined and considered in the controlling of the stepper motor.

An X-ray system according to an alternative or additional aspect of the invention comprises: a first structure comprising a housing or holder, in particular a so-called bucky, which is configured to accommodate an X-ray detector; a second structure comprising an X-ray table or X-ray stand, in particular a wall stand, on which the holder or housing accommodating the X-ray detector is moveably mounted; a stepper motor comprising a rotor shaft which is coupled with a threaded spindle, the threaded spindle having a thread pitch; and a coupling element comprising a threaded nut which is in mesh with the threaded spindle, wherein the coupling element is, in particular fixedly, mounted at the first structure or the second structure, and the stepper motor is, in particular fixedly, mounted at the second structure or the first structure, respectively, such that by means of rotating the rotor shaft of the stepper motor the first structure, in particular the housing or holder, is moved relative to the second structure, in particular the X-ray table or X-ray stand. Preferably, the relative movement between the first and second structure corresponds to a translation, i.e. a linear movement.

According to a preferred embodiment, the threaded spindle has a thread pitch between approximately 30 mm and 70 mm per revolution, preferably between 40 mm and 60 mm per revolution, most preferably between 45 mm and 55 mm per revolution. In a particularly preferred embodiment, the threaded pitch is approximately 50 mm per revolution. According to an alternative or additional embodiment, the spindle has a diameter between 5 mm and 20 mm, in particular between 8 mm and 15 mm, and most preferably of approximately 10 mm.

Alternatively or additionally, the threaded spindle is not self-locking, i.e. the threaded spindle does not inhibit a movement of the first structure relative to the second structure in cases where the rotor shaft is not driven by the motor. In particular, the thread pitch of the threaded spindle is chosen such that the first structure, in particular the housing or holder, can be manually moved relative to the second structure, in particular the X-ray table or X-ray stand, by applying a manual force of less than approximately 10 N to the first structure. Preferably, the thread pitch is larger than 30 mm per revolution.

By means of the alternative or additional aspect of the invention and/or the preferred embodiments mentioned above, the first structure, e.g. the bucky which accommodates the X-ray detector, can be exactly positioned relative to the second structure, e.g. the X-ray table or X-ray wall stand, by driving the threaded spindle with the stepper motor. Moreover, the first structure can be easily moved relative to the second structure just by exerting a manual force to the first structure, for example in cases in which the stepper motor is not energized or decoupled from the rotor shaft so that the rotor shaft and the threaded spindle are not driven by the stepper motor. Last but not least, in cases in which the stepper motor is energized and coupled with the rotor shaft, the first structure can be moved relative to the second structure with high speed. All in all, exact and fast positioning as well as easy manual positioning of the first structure, e.g. a bucky accommodating the X-ray detector, relative to the second structure, e.g. an X-ray table, is enabled.

An X-ray system according to another alternative or additional aspect of the invention comprises: a first structure, on which an X-ray source and/or an X-ray detector is provided; a second structure; at least one stepper motor, which is configured to move the first structure relative to the second structure, the stepper motor comprising a rotor shaft which is coupled with the first structure and/or the second structure; at least one rotary encoder, which is coupled with the rotor shaft of the stepper motor and configured to generate an encoder signal in accordance with the angular position of the rotor shaft; and a control unit, which is configured to determine, based on the encoder signal, an absolute position value corresponding to an absolute position of the first structure relative to the second structure, to compare the absolute position value with at least one predefined detent position value corresponding to a position in which the first structure shall be locked relative to the second structure, and to control the stepper motor to rotate the rotor shaft such that the first structure is moved relatively to the second structure until the absolute position value corresponds to the at least one pre-defined detent position value.

Preferably, the control unit is configured to control the stepper motor to brake and/or stop rotating the rotor shaft and/or to retain the rotor shaft in an angular position in the case that the position value corresponds to the at least one predefined detent position value.

Alternatively or additionally, the control unit is configured to control the stepper motor to rotate the rotor shaft such that the correspondence of the absolute position value and the at least one pre-defined detent position value is maintained. Preferably, correspondence of the absolute position value and the at least one pre-defined detent position value is maintained, even if an external manual force is exerted on the first structure.

Alternatively or additionally, the control unit is configured to control the stepper motor to accelerate or decelerate or brake the rotation of the rotor shaft in the case that the absolute position value is close to and/or within a defined range around the at least one pre-defined detent position value. Alternatively or additionally, the control unit is configured to control, depending on the current velocity, the stepper motor to decelerate or accelerate the rotation of the rotor shaft when approaching and/or diverging from the at least one pre-defined detent position value. Preferably, the control unit is configured to perform a small accurately timed positioning to reach the pre-defined detent position with defined accuracy.

By means of the alternative or additional aspect of the invention and/or the preferred embodiments mentioned above, the first structure can be moved to and locked or retained in pre-defined detent positions or lock-in positions without the need of additional mechanical, electromechanical or electromagnetic means, for example V-like grooves into which the first structure has to engage in order to be retained in a lock-in position. In this way, the desired detent positions can be easily established and/or changed by pre-defining according detent position values without the need of shifting or installing mechanical, electromechanical or electromagnetic means, like V-like grooves. Moreover, the first structure can be moved into the detent positions in a very accurate and smooth way, both in the case that the relative movement is driven by the stepper motor only, and in the case that the relative movement is initiated and/or powered by exerting a manual force on the first structure and supported by the stepper motor drive, as described above in connection with an aspect of the invention.

According to yet another preferred embodiment, the control unit is configured to control the stepper motor such that, if a mutual velocity of the first and second structure at a detent position is less then a critical threshold, the relative movement is stopped and the reached detent position is held. Alternatively or additionally, the control unit is configured to control the stepper motor such that, if a mutual velocity of the first and second structure is higher then a critical threshold, the movement is continued with a "jiggle" effect providing a haptic feedback to the user having his hand or hands on the first structure. In this way, the user gets an intuitively comprehensible feedback that a detent position has been touched.

Further advantages, aspects and examples and alternatives of the present invention will be apparent from the description of following figures, which show:
- Fig. 1: examples of X-ray systems in a perspective view;
- Fig. 2: a top view on a section of an example of an X-ray system;
- Fig. 3: a side view on the example of Fig. 2;
- Fig. 4: a side view on the third X-ray system shown in Fig. 1;
- Fig. 5: a schematic representation of an example of a control unit;
- Fig. 6: a flow chart which illustrates steps of an example of a method for manually operating an X-ray system; and
- Fig. 7: a top view on another example of an X-ray system.

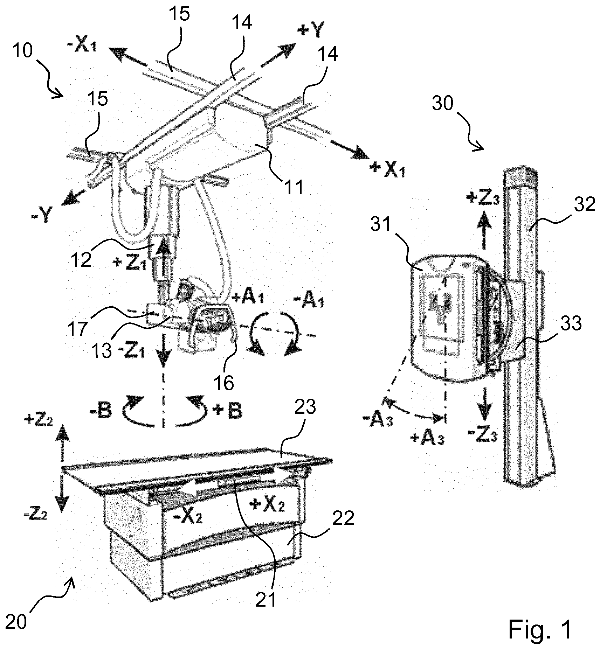

Figure 1 shows examples of X-ray systems in a perspective view. A first X-ray system 10 comprises a first structure 11, for example a carriage, on which a telescope arm 12 is mounted. At the lower end of the telescope arm 12 a so-called gooseneck 17 is mounted on which a housing 13 accommodating an X-ray source is provided. The first structure 11 is moveably mounted on a second structure, which comprises first guide rails 14 and second guide rails 15 which are fixed on the ceiling. On the housing 13 a tube head 28 with a handle 16 is provided which can be grasped by a user to move the housing 13 including the X-ray source into a desired position.

Preferably, the housing 13 is pivotably mounted on the telescope arm 12 such that the housing 13 can be rotated in direction +A1 and -A1 around a horizontal axis and/or in direction +B and -B along a vertical axis. By means of the telescope arm 12 the housing 13 can be moved along the vertical axis in direction +Z1 and -Z1. Likewise, by applying an according force via handle 16, the carriage of the first structure 11 can be moved in direction +Y and -Y along the first guide rails 14 and/or in direction +X1 and -X1 along the second guide rails 15.

A second X-ray system 20 comprises, as a second structure, an X-ray table 23 which is provided on a table base 22. An X-ray detector and/or an X-ray cassette (not shown) accommodating an X-ray sensitive medium or film can be inserted into a so-called X-ray bucky 21, as a first structure, which is provided under the X-ray table 23. The X-ray bucky 21 is moveably mounted on the X-ray table 23 and/or on the table base 22 such that it can be linearly moved in direction +X2 and -X2 in parallel to the plane of the X-ray table 23.

A third X-ray system 30 comprises, as a first structure, an X-ray bucky or holder 31 which is configured to accommodate an X-ray detector and/or an X-ray cassette (not shown). The first structure 31 is pivotably mounted on a carriage 33. The carriage 33 is slidably mounted on a vertical pillar 32 which is fixed on the floor or on a wall. Preferably, the first structure 31 can be rotated around a horizontal axis in direction -A3 and +A3 relative to the carriage 33, which is the second structure in this case. Additionally or alternatively, the first structure 31 can be linearly moved relative to the pillar 32, which is the second structure in this case, along vertical direction +Z3 and -Z3. By exerting a manual force and/or torque on the first structure 31, a movement of the first structure 31 relative to the second structure, i.e. the pillar 32 and/or the carriage 33, respectively, can be initiated.

Apart from the aforementioned examples of first and second structures, further combinations of relatively moveable first and second structures are possible. For example, first guide rails 14 (first structure) that can be translated relative to second guide rails 15 (second structure), gooseneck 17 (first structure) can be rotated and/or translated relative to carriage 11 (second structure) and X-ray tube 13 with tube head 28 (first structure) can be rotated relative to gooseneck 17 (second structure). Therefore, the following elucidations given in relation to particular examples of first and second structures also apply accordingly to any other example of first and second structures.

According to a preferred aspect of the invention, a movement of the first structure, e.g. carriage 11, X-ray bucky 21 or X-ray bucky 31, of the first, second or third X-ray system 10, 20 or 30, respectively, relative to respective second structure, e.g. first guide rails 14 and/or second guide rails 15, X-ray table 22 and 23 or pillar 32, respectively, can be initiated and/or actuated by exerting a manual force and/or torque on the first structure, for example by grasping the handle 16 and exerting a force along the directions shown in connection with the first X-ray system 10, by exerting a manual force on a handle (not shown) provided on the X-ray bucky 21 along the X direction shown in connection with the second X-ray system 20 or by exerting a manual force on the first structure 31, i.e. the X-ray bucky or holder, along the directions shown in connection with the third X-ray system 30.

According to a particularly preferred aspect of the invention, the relative movement between the first structure, on the one hand, and the second structure, on the other hand, is supported by a stepper motor drive in accordance with direction and/or magnitude of the manual force that is exerted on the first structure, respectively. This will be elucidated in more detail in the following.

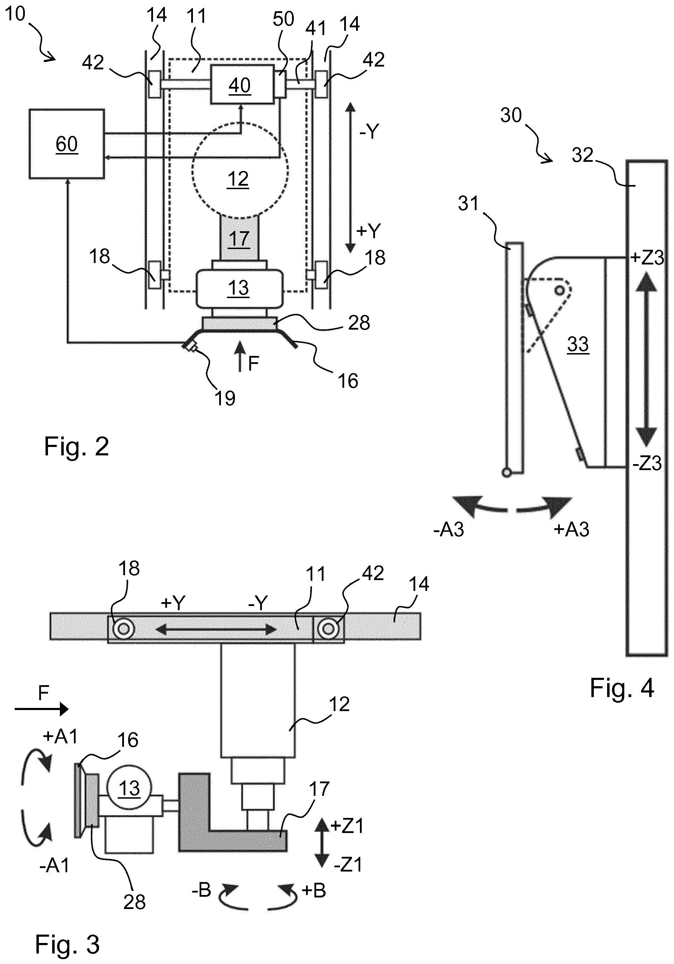

Figure 2 shows a top view on a section of an example of the first X-ray system 10. In the present example, the first structure 11, in particular the carriage, is provided with rollers 18 which are guided in first guide rails 14. Moreover, a stepper motor 40 is provided comprising a rotor shaft 41 on which driving wheels 42 are provided. The driving wheels 42 are coupled with both guide rails 14, for example by frictional connection and/or form-fit, such that when the driving wheels 42 are rotated by the stepper motor 40, the carriage of the first structure 11 is moved along the first guide rails 14 in parallel to direction +Y and -Y.

Preferably, the rotor shaft 41 of the stepper motor 40 is coupled with an incremental rotary encoder 50 which is configured to generate an encoder signal in accordance with the change of the angular position of the rotor shaft 41. If a user applies a manual force F on the first structure 11, for example on the handle 16 which is mechanically coupled to the first structure 11 via housing 13, gooseneck 17 and telescope arm 12, the first structure 11 is slightly moved along the direction of the applied force F, i.e. in direction -Y, whereby the driving wheel 42 together with the rotor shaft 41 of the stepper motor 40 are slightly rotated so that the current angular position of the rotor shaft 41 deviates from its original position. The according encoder signal generated by the incremental rotary encoder 50 is guided to a control unit 60, where a position deviation signal is determined that characterizes the deviation of the current angular position of the rotor shaft 41 from the initial angular position.

According to another preferred embodiment, a so-called release button 19 can be provided, for example on or near the handle 16. Preferably, the release button 19 is connected with the control unit 60, which is configured to control the stepper motor 40 only in case that the release button 19 has been or is being pressed by the user. In this way, an unintentional initiation and/or support of a relative movement of the first and second structure 11 and 14, respectively, can be avoided.

Preferably, the control unit 60 is further configured to control the stepper motor 40 to rotate the rotor shaft 41 together with the driving wheel 42, preferably subject to an according release button 19 signal, dependent on the determined position deviation signal in such a way that the stepper motor 40 supports a movement of the first structure 11 relative to the first guide rails 14 of the second structure in accordance with magnitude and/or direction of the exerted external force F. For example, if the exerted manual force F is directed in -Y direction and exceeds a defined threshold, a movement of the first structure 11 relative to the guide rails 14 of the second structure is supported in -Y direction.

Fig. 3 shows a side view on the example of Fig. 2. As apparent from this representation, the carriage 11 is slidably mounted on first guide rail 14 by means of rollers 18 and driving wheels 42. On carriage 11a telescope arm 12 is provided at the end of which gooseneck 17 is mounted. On gooseneck 17 a housing 13 accommodating the X-ray source is provided. Tube head 28 together with housing 13 (X-ray source) and gooseneck 17 can be rotated relative to the telescope arm 12 and/or the carriage 11 in direction +B and -B around a vertical axis and translated in vertical direction +Z and -Z. Tube head 28 together with X-ray source 13 can be rotated relative to gooseneck 17 in direction +A1 and -A1 around a horizontal axis.

Similarly to the support of the movement of the carriage 11 relative to the first guide rail 14 upon exertion of a manual force F on the handle 16 by accordingly controlled motor 40, a movement of tube head 28 together with housing 13 (X-ray source) and gooseneck 17 relative to carriage 11 in vertical direction +Z and -Z can also be supported by a motor (not shown) which is accordingly controlled by the control unit to support a relative movement of these components upon exerting a manual force and/or torque on handle 16. Alternatively, both the rotatory motion of housing 13 with tube head 28 and handle 16 in direction +A1 and -A1 around a horizontal axis and in direction +B and -B around a vertical axis may be driven and/or supported by a motor (not shown) in accordance with a manual force and/or torque applied on handle 16.

Fig. 4 shows a side view on the third X-ray system 30 shown in Fig. 1. As apparent from this representation, X-ray bucky 31 is pivotably mounted on carriage 33 which is slidably mounted on vertical pillar 32. Preferably, both the motion of X-ray bucky 31 relative to carriage 33 and of carriage 33 relative to pillar 32 may be driven and/or supported by a motor (not shown) in accordance with a manual force and/or torque applied on the X-ray bucky 31.

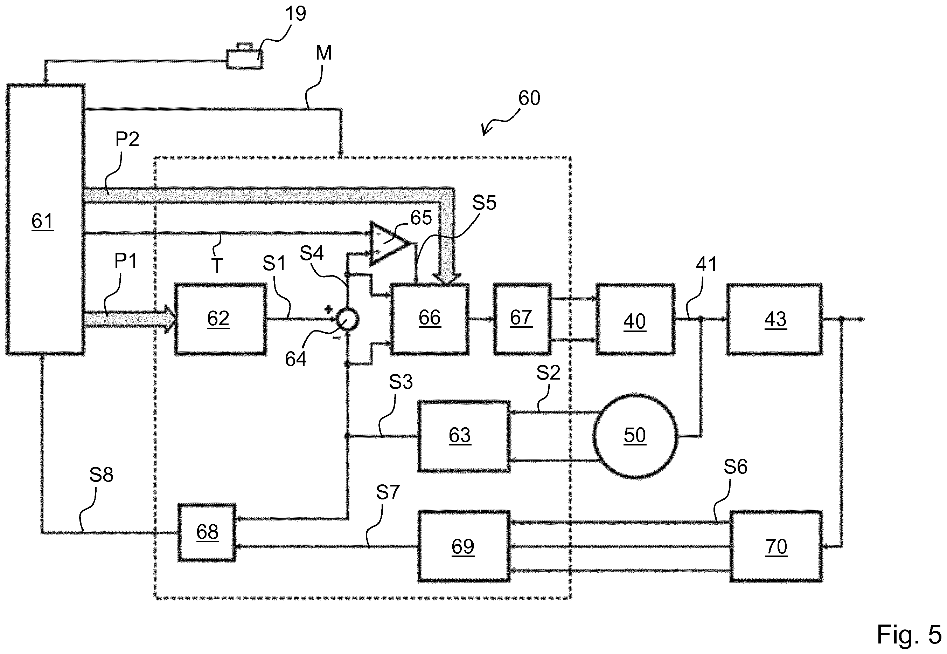

Figure 5 shows a schematic representation of an example of a control unit 60 which is configured to control stepper motor 40 to rotate the rotor shaft 41 by which one or more mechanical components 43, e.g. first structure 11, 13 and 16, 21 or 31 (see Fig. 1 to 4), are moved.

A high level control element 61 is configured to specify movement parameters P1 which are fed to a position generator 62 which generates a position signal S1 corresponding to a desired position of the rotor shaft 41 of stepper motor 40.

The control element 61 is further configured to specify a threshold value T which preferably corresponds to a predefined angular position deviation of the rotor shaft 41. By specifying the threshold value T, a sensitivity of the stepper motor drive of the mechanical components 43, e.g. the first structure 11, with respect to a force F exerted on the mechanical components 43 can be defined.

Preferably, the control element 61 is further configured to pre-define vector control parameters P2 as well as to pre-define an operation mode M of the stepper motor drive, for example a speed/positioning mode, a holding/break mode, a force-sensing mode, a support mode and/or an idle mode.

The current angular position of the rotor shaft 41 of the stepper motor 40 is detected by an incremental rotary encoder 50 and converted into according encoder signals S2 which are forwarded to a position counter element 63 which is configured to determine a position signal S3, corresponding to the current or real angular position of the rotor shaft 41. The position signal S3 is forwarded to a vector control element 66, a difference operator 64 and a multiplexer 68.

The difference operator 64 is configured to compare the position signal S3 corresponding to the current angular position of the rotor shaft 41 with the position signal S1 corresponding to the desired angular position of the rotor shaft 41. In dependence on this difference, a position error signal S4 is generated and forwarded to the vector control element 66 and to a comparator element 65, where the position error signal S4, also referred to as angular position deviation, is compared with the threshold value T corresponding to the pre-defined minimum angular position deviation of the rotor shaft 41. The output signal S5 of the comparator element 65 is forwarded to the vector control element 66, which is configured to control a stepper motor driver element 67 to energize and control the stepper motor 40 based on signals S3 and/or S4 and/or S5 and/or the vector control parameters P2.

Preferably, the aforementioned control of the motor 40 takes into account whether release button 19 is being pressed or has been pressed by a user. Preferably, the control element 61 is configured to examine whether an according release button signal is present and, in the affirmative, to initiate or enable an appropriate control mode.

Optionally, an absolute encoder 70 may be provided which is configured to detect an absolute position of at least one of the mechanical components 43, in particular an absolute position of the first structure 11,21 or 31 (see Fig. 1 to 4), and to generate according absolute position signals S6 which are forwarded to a position register 69 that is configured to determine a position signal S7 corresponding to the current or real absolute position of the mechanical components 43.

The signal S7 is forwarded to the multiplexer 68, which is configured to switch between signals S3 and S7 corresponding to the current angular position of the rotor shaft 41 and the current absolute position of the mechanical components 43, respectively. The output signal S8 is forwarded to the high level control element 61.

Preferably, the control unit 60 is configured to control the operation of the stepper motor 40 based on the respective operation mode M which is selected by the high level control element 61. This will be illustrated in more detail in the following.

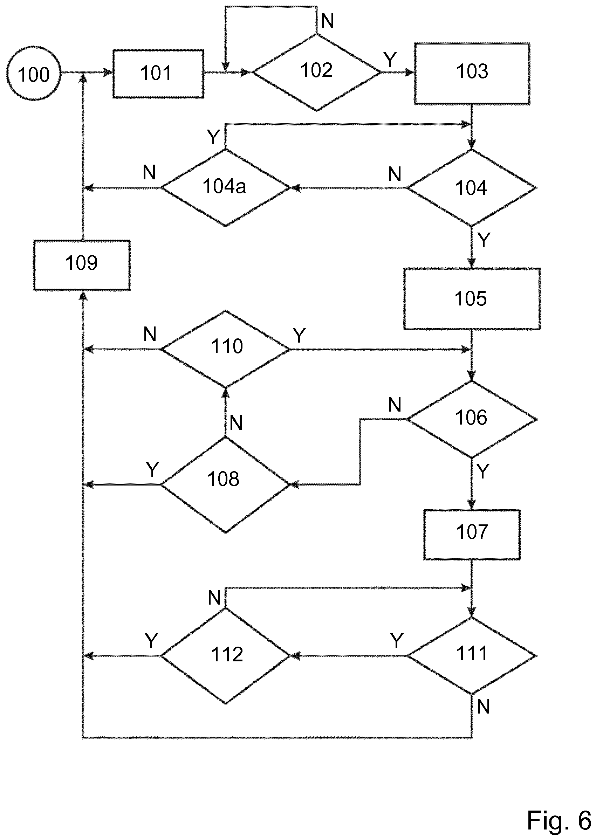

Figure 6 shows a flow chart which illustrates steps (100) to (112) of an example of a method for operating an X-ray system. After starting (100) the system, a holding mode is activated (101) in which an initial position of the first structure relative to the second structure of the X-ray system is maintained.

After the holding mode has been activated, it is checked whether the release button 19 (see figure 2) has been or is being pressed (102). In case of affirmation (Y), a force-sensing mode is activated (103), and it is checked whether an external force exerted by a user on the first structure of the system exceeds a pre-defined threshold value (104).

In the case that the position deviation signal does not exceed the pre-defined threshold value (N), it is checked again whether the release button 19 is pressed (104a). In the affirmative (Y), step (104) is repeated. Otherwise (N), the force sensing mode is deactivated and only the holding mode remains activated (101).

In the case that the position deviation signal exceeds the pre-defined threshold value (Y), a support mode is activated (105), in which the stepper motor is controlled such that it supports a movement of the first structure, on which the external force is exerted, relative to the second structure in accordance with the exerted external force. Preferably, the stepper motor is controlled to generate the supporting torque with a defined magnitude, based on the position deviation signal, which corresponds to the direction of the exerted external force.

In the case that a deceleration of a relative movement between the first and second structure is detected (Y) in step (106) following step (105), an idle mode is activated (107), in which the support of the relative movement by the stepper motor is deactivated.

In the case that in step (106) no deceleration of the movement of the first structure relative to the second structure is detected (N), it is checked (108) whether a pre-defined event has occurred, for example whether a virtual stop or detent position has been reached or an emergency stop is required. In case of affirmation (Y), a break mode is activated (109), in which the movement of the first structure relative to the second structure is decelerated and/or stopped. In the case that in step (108) no particular event is detected (N), it is checked, whether the release button is being pressed (110). In case of affirmation (Y), the support mode is maintained (105) and it is checked again whether a deceleration has been detected (106). In case that step (110) yields that the release button is no longer pressed (N), the break mode is activated (109).

Upon detection of a deceleration (106) and subsequent activation of the idle mode (107), it is checked whether the release button is being pressed (111). In case of affirmation (Y), it is checked again whether a pre-defined event has been detected (112), for example whether a virtual stop or detent position has been reached or an emergency stop is required. In the affirmative (Y), the break mode (109) is activated. Otherwise (N), it is checked again, whether the release button is being pressed (111) and so forth. In the case that step (111) yields that the release button is no longer pressed (N) the break mode is activated (109).

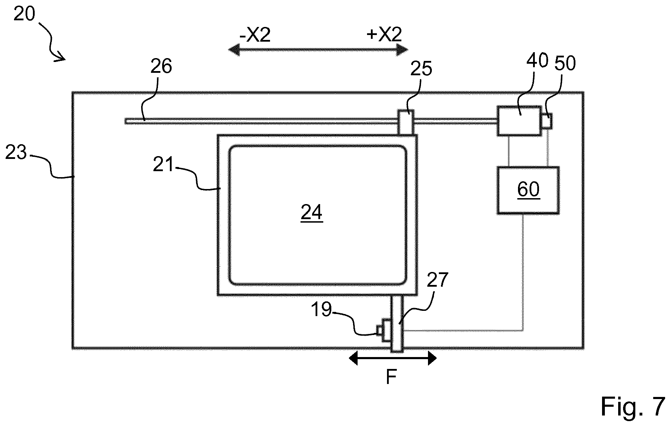

Figure 7 shows a top view of another example of the second X-ray system 20 shown in Figure 1. Below table top 23, an X-ray bucky 21 is provided which is configured to accommodate an X-ray detector or X-ray cassette 24. The X-ray bucky 21 is moveably mounted so that it can be linearly moved in direction +X2 and -X2 as indicated by the double arrow.

In the present example, the X-ray bucky 21 is coupled with a screw nut 25 that is provided on a threaded spindle 26 which is coupled with the rotor shaft (not shown) of stepper motor 40.

Preferably, an incremental rotary encoder 50 is provided for determining a change of the angular position of the rotor shaft and/or the threaded spindle 26. According encoder signals are evaluated in control unit 60, which is also configured to control the stepper motor 40 based on the encoder signals.

Preferably, the control unit 60 is configured to control the stepper motor 40 to rotate the threaded spindle 26 such that the stepper motor 40 supports a movement of the X-ray bucky 21 relative to the table top 23 in accordance with an external force F which is exerted on the X-ray bucky 21, for example via a handle 27 that is provided on the X-ray bucky 21. In this respect, the above elucidations apply accordingly. Preferably, the control unit is configured such that the stepper motor 40 supports the movement of bucky 21 only in case that release button 19 has been or is being pressed.

Alternatively or additionally, the threaded spindle 26 has a thread pitch between approximately 30 mm and 70 mm per revolution, preferably between 40 mm and 60 mm per revolution. Due to such a relatively large thread pitch the X-ray bucky 21 can be both reliably and quickly positioned relative to the table top 23. Moreover, the X-ray bucky 21 can be easily moved and/or positioned relative to the table top 23 just by exerting a manual force to the X-ray bucky 21, which is of particular advantage in cases where the stepper motor 40 is not energized so that the rotor shaft and the threaded spindle 26 are not driven by the stepper motor 40. Last but not least, the threaded spindle 26 having a relatively high thread pitch allows for a precise and reliable detection of changes of the current angular position of the threaded spindle 26 and a favorable response characteristic of the servo motor support of the relative movement upon pushing release button 19 and exertion of an external force F on the X-ray bucky 21, e.g. via handle 27.

## Claims

1. An X-ray system comprising
- a first structure (11, 17; 21; 31), on which an X-ray source and/or an X-ray detector is provided,
- a second structure (14, 15; 22; 32),
- at least one stepper motor (40), which is configured to move the first structure (11, 17; 21; 31) relative to the second structure (14, 15; 22; 32), the stepper motor (40) comprising a rotor shaft (41) which is coupled with the first structure (11, 17; 21; 31) such that when a user exerts an external force (F) on the first structure (11, 17; 21; 31) an angular position of the rotor shaft (41) is changed in accordance with the exerted external force (F),
- at least one incremental rotary encoder (50), which is coupled with the rotor shaft (41) of the stepper motor (40) and configured to generate an encoder signal (S2) in accordance with the change of the angular position of the rotor shaft (41); and
- a control unit (60), which is configured to determine, based on the encoder signal (S2), a position deviation signal (S4) characterizing a deviation of the angular position (S3) of the rotor shaft (41) from a reference angular position (S1) of the rotor shaft (41) and to control the stepper motor (40) to rotate the rotor shaft (41) dependent on the position deviation signal (S4) such that the stepper motor (40) supports a movement of the first structure (11, 17; 21; 31) relative to the second structure (14, 15; 22; 32) in accordance with the exerted external force (F).

2. The X-ray system according to claim 1, wherein the control unit is configured to determine whether the position deviation signal exceeds a predefined value and, if the position deviation signal exceeds the predefined value, to control the stepper motor to rotate the rotor shaft dependent on the position deviation signal such that the stepper motor supports a movement of the first structure relative to the second structure in accordance with the exerted external force.

3. The X-ray system according to claim 1 or 2, wherein the control unit is configured to control the stepper motor to rotate the rotor shaft dependent on the sign of the position deviation signal such that the stepper motor supports a movement of the first structure relative to the second structure in accordance with a direction of the exerted external force.

4. The X-ray system according to any of the preceding claims, wherein the control unit is configured
- to determine, based on the encoder signal generated during the support of the movement of the first structure relative to the second structure, an angular velocity of the rotor shaft and whether the angular velocity of the rotor shaft exceeds or falls below a predefined value and,
- if the angular velocity of the rotor shaft exceeds or falls below the predefined value, to control the stepper motor to stop supporting the movement of the first structure relative to the second structure.

5. The X-ray system according to any of the preceding claims, wherein the control unit is configured
- to determine, based on the encoder signal generated during the support of the movement of the first structure relative to the second structure, an angular velocity of the rotor shaft and whether the angular velocity of the rotor shaft exhibits a decrease that exceeds a predefined value and,
- if the decrease of the velocity of the rotor shaft exceeds the predefined value, to control the stepper motor to stop supporting the movement of the first structure relative to the second structure.

6. The X-ray system according to any of the preceding claims, wherein the control unit is configured to generate a reference position signal corresponding to an initial reference angular position of the rotor shaft.

7. A method for operating an X-ray system, the X-ray system comprising a first structure (11, 17; 21; 31), on which an X-ray source and/or an X-ray detector is provided, a second structure (14, 15; 22; 32) and at least one stepper motor (40), the stepper motor (40) comprising a rotor shaft (41) which is coupled with the first structure (11, 17; 21; 31), said method comprising the following steps:
- changing an angular position of the rotor shaft (41) in accordance with an external force (F), which is exerted on the first structure (11, 17; 21; 31) by a user,
- generating an encoder signal (S2) by means of an incremental rotary encoder (50), which is coupled with the rotor shaft (41) of the stepper motor (40), in accordance with the change of the angular position of the rotor shaft (41),
- determining, based on the encoder signal (S2), a position deviation signal (S4) characterizing a deviation of the angular position (S3) of the rotor shaft (41) from a reference angular position (S1) of the rotor shaft (41), and
- controlling the stepper motor (40) to rotate the rotor shaft (41) dependent on the position deviation signal (S4) such that the stepper motor (40) supports a movement of the first structure (11, 17; 21; 31) relative to the second structure (14, 15; 22; 32) in accordance with the exerted external force (F).

## Patentansprüche

1. Ein Röntgensystem, umfassend
- eine erste Struktur (11, 17; 21; 31), auf der eine Röntgenquelle und/oder ein Röntgendetektor angeordnet ist (sind),
- eine zweite Struktur (14, 15; 22; 32),
- mindestens einen Schrittmotor (40), der so konfiguriert ist, dass er die erste Struktur (11, 17; 21; 31) relativ zur zweiten Struktur (14, 15; 22; 32) bewegt, wobei der Schrittmotor (40) eine Rotorwelle (41) umfasst, die derart mit der ersten Struktur (11, 17; 21; 31) gekoppelt ist, dass, wenn ein Benutzer eine äußere Kraft (F) auf die erste Struktur (11, 17; 21; 31) ausübt, eine Winkelposition der Rotorwelle (41) in Übereinstimmung mit der ausgeübten äußeren Kraft (F) geändert wird,
- mindestens einen Inkrementaldrehgeber (50), der mit der Rotorwelle (41) des Schrittmotors (40) gekoppelt ist und derart konfiguriert ist, dass er in Übereinstimmung mit der Änderung der Winkelposition der Rotorwelle (41) ein Drehgebersignal (S2) erzeugt; und
- eine Steuereinheit (60), die derart konfiguriert ist, dass sie auf der Basis des Drehgebersignals (S2) ein Positionsabweichungssignal (S4), das eine Abweichung der Winkelposition (S3) der Rotorwelle (41) von einer Bezugswinkelposition (S1) der Rotorwelle (41) kennzeichnet, bestimmt und den Schrittmotor (40) so steuert, dass er die Rotorwelle (41) in Abhängigkeit des Positionsabweichungssignals (S4) derart dreht, dass der Schrittmotor (40) eine relativ zur zweiten Struktur (14, 15; 22; 32) verlaufende Bewegung der erste Struktur (11, 17; 21; 31) in Übereinstimmung mit der ausgeübten äußeren Kraft (F) unterstützt.

2. Das Röntgensystem nach Anspruch 1, wobei die Steuereinheit derart konfiguriert ist, dass sie bestimmt, ob das Positionsabweichungssignal einen vordefinierten Wert übersteigt, und, falls das Positionsabweichungssignal den vordefinierten Wert übersteigt, den Schrittmotor so steuert, dass er die Rotorwelle in Abhängigkeit des Positionsabweichungssignals so dreht, dass der Schrittmotor eine relativ zur zweiten Struktur verlaufende Bewegung der ersten Struktur in Übereinstimmung mit der ausgeübten äußeren Kraft unterstützt.

3. Das Röntgensystem nach Anspruch 1 oder 2, wobei die Steuereinheit derart konfiguriert ist, dass sie den Schrittmotor so steuert, dass er die Rotorwelle in Abhängigkeit des Zeichens des Positionsabweichungssignals so dreht, dass der Schrittmotor eine relativ zur zweiten Struktur verlaufende Bewegung der ersten Struktur in Übereinstimmung mit einer Richtung der ausgeübten äußeren Kraft unterstützt.

4. Das Röntgensystem nach einem der vorstehenden Ansprüche, wobei die Steuereinheit derart konfiguriert ist,
- dass sie auf der Basis des Drehgebersignals, das während der Unterstützung der relativ zur zweiten Struktur verlaufenden Bewegung der ersten Struktur erzeugt wird, eine Winkelgeschwindigkeit der Rotorwelle bestimmt und bestimmt, ob die Winkelgeschwindigkeit der Rotorwelle oberhalb oder unterhalb eines vordefinierten Werts liegt, und
- falls die Winkelgeschwindigkeit der Rotorwelle oberhalb oder unterhalb des vordefinierten Werts liegt, den Schrittmotor so steuert, dass er die Unterstützung der relativ zur zweiten Struktur verlaufenden Bewegung der ersten Struktur stoppt.

5. Das Röntgensystem nach einem der vorstehenden Ansprüche, wobei die Steuereinheit derart konfiguriert ist
- dass sie auf der Basis des Drehgebersignals, das während der Unterstützung der relativ zur zweiten Struktur verlaufenden Bewegung der ersten Struktur erzeugt wird, eine Winkelgeschwindigkeit der Rotorwelle bestimmt und bestimmt, ob die Winkelgeschwindigkeit der Rotorwelle eine Abnahme, die einen vordefinierten Wert übersteigt, aufweist, und
- falls die Abnahme der Geschwindigkeit der Rotorwelle den vordefinierten Wert übersteigt, den Schrittmotor so steuert, dass er die Unterstützung der relativ zur zweiten Struktur verlaufenden Bewegung der ersten Struktur stoppt.

6. Das Röntgensystem nach einem der vorstehenden Anspruche, wobei die Steuereinheit derart konfiguriert ist, dass sie ein Bezugspositionssignal, das einer Bezugswinkelanfangsposition der Rotorwelle entspricht, erzeugt.

7. Ein Verfahren zum Betrieb eines Röntgensystezns, wobei das Röntgensystem eine erste Struktur (11, 17; 21; 31), auf der eine Röntgenquelle und/oder ein Röntgendetektor angeordnet ist (sind), eine zweite Struktur (14, 15; 22; 32) und mindestens einen Schrittmotor (40) umfasst, wobei der Schrittmotor (40) eine mit der ersten Struktur (11, 17; 21; 31) gekoppelte Rotorwelle (41) umfasst, wobei das Verfahren die folgenden Schritte umfasst:
- Änderung einer Winkelposition der Rotorwelle (41) in Übereinstimmung mit einer äußeren Kraft (F), die von einem Benutzer auf die erste Struktur (11, 17; 21; 31) ausgeübt wird,
- Erzeugung eines Drehgebersignals (S2) mittels eines mit der Rotorwelle (41) des Schrittmotors (40) gekoppelten Inkrementaldrehgebers (50) in Übereinstimmung mit der Änderung der Winkelposition der Rotorwelle (41),
- Bestimmen, auf der Basis des Drehgebersignals (S2), eines Positicansabweichungssignals (S4), das eine Abweichung der Winkelposition (S3) der Rotorwelle (41) von einer Bezugswinkelposition (S1) der Rotorwelle (41) kennzeichnet, und
- Steuern des Schrittmotors (40), um die Rotorwelle (41) in Abhängigkeit des Positionsabweichungssignals (S4) so zu drehen, dass der Schrittmotor (40) eine relativ zur zweiten Struktur (14, 15; 22; 32) verlaufende Bewegung der ersten Struktur (11, 17; 21; 31) in Übereinstimmung mit der ausgeübten äußeren Kraft (F) unterstützt.

## Revendications

1. Système à rayons X comprenant
- une première structure (11, 17; 21; 31) sur laquelle est (sont) disposé(e)(s) une source de rayons X et/ou un détecteur de rayons X,
- une deuxième structure (14, 15; 22; 32),
- au moins un moteur pas à pas (40) configuré de façon à déplacer la première structure (11, 17; 21; 31) de façon relative à la deuxième structure (14, 15; 22; 32), ledit moteur pas à pas (40) comprenant un arbre de rotor (41) couplé à la première structure (11, 17; 21; 31) de façon à ce que, lorsqu'un utilisateur exerce une force externe (F) sur la première structure (11, 17; 21; 31), une position angulaire de l'arbre de rotor (41) soit modifiée conformément à la force externe exercée (F),
- au moins un codeur rotatif incrémental (50) couplé à l'arbre de rotor (41) du moteur pas à pas (40) et configuré de façon à générer un signal de codeur (S2) conformément à la modification de la position angulaire de l'arbre de rotor (41); et
- une unité de commande (60) configurée de façon à déterminer, sur la base du signal de codeur (S2), un signal de déviation positionnelle (S4) caractérisant une déviation de la position angulaire (S3) de l'arbre de rotor (41) par rapport à une position angulaire de référence (S1) de l'arbre de rotor (41) et à commander le moteur pas à pas (40) de façon à ce que ce dernier fasse tourner l'arbre de rotor (41) en fonction du signal de déviation positionnelle (S4) de façon à ce que le moteur pas à pas (40) supporte un déplacement de la première structure (11, 17; 21; 31) qui est relatif à la deuxième structure (14, 15; 22; 32) et conforme à la force externe exercée (F).

2. Système à rayons X selon la revendication 1, **caractérisé en ce que** l'unité de commande est configurée de façon à déterminer si le signal de déviation positionnelle dépasse une valeur prédéfinie et, si le signal de déviation positionnelle dépasse la valeur prédéfinie, à commander le moteur pas à pas de façon à ce qu'il fasse tourner l'arbre de rotor en fonction du signal de déviation positionnelle de façon à ce que le moteur pas à pas supporte un déplacement de la première structure qui est relatif à la deuxième structure et conforme à la force externe exercée.

3. Système à rayons X selon la revendication 1. ou 2, **caractérisé en ce que** l'unité de commande est configurée de façon à commander le moteur pas à pas de façon à ce que ce dernier fasse tourner l'arbre de rotor en fonction du signe du signal de déviation positionnelle de façon à ce que le moteur pas à pas supporte un déplacement de la première structure qui est relatif à la deuxième structure et conforme à un sens de la force externe exercée.

4. Système à rayons X selon l'une quelconque des revendications précédentes, **caractérisé en ce que** l'unité de commande est configurée de façon à
- déterminer, sur la base du signal de codeur généré lors du support du déplacement de la première structure qui est relatif à la deuxième structure, une vitesse angulaire de l'arbre de rotor et à déterminer si la vitesse angulaire de l'arbre de rotor est supérieure ou inférieure à une valeur prédéfinie et,
- si la vitesse angulaire de l'arbre de rotor est supérieure ou inférieure à la valeur prédéfinie, à commander le moteur pas à pas de façon à ce qu'il arrête le support du déplacement de la première structure qui est relatif à la deuxième structure.

5. Système à rayons X selon l'une quelconque des revendications précédentes, **caractérisé en ce que** l'unité de commande est configurée de façon à
- déterminer, sur la base du signal de codeur généré lors du support du déplacement de la première structure qui est relatif à la deuxième structure, une vitesse angulaire de l'arbre de rotor et à déterminer si la vitesse angulaire de l'arbre de rotor présente une baisse qui dépasse une valeur prédéfinie et,
- si la baisse de la vitesse de l'arbre de rotor dépasse la valeur prédéfinie, à commander le moteur pas à pas de façon à ce qu'il arrête le support du déplacement de la première structure qui est relatif à la deuxième structure.

6. Système à rayons X selon l'une quelconque des revendications précédentes, **caractérisé en ce que** l'unité de commande est configurée de façon à générer un signal de position de référence correspondant à une position angulaire de référence initiale de l'arbre de rotor.

7. Procédé pour faire fonctionner un système à rayons X, ledit système à rayons X comprenant une première structure (11, 17; 21; 31) sur laquelle est (sont) disposé (e) (s) une source de rayons X et/ou un détecteur de rayons X, une deuxième structure (14, 15; 22; 32) et au moins un moteur pas à pas (40), ledit moteur pas à pas (40) comprenant un arbre de rotor (41) couplé à la première structure (11, 17; 21; 31), ledit procédé comprenant les étapes consistant à:
- modifier une position angulaire de l'arbre de rotor (41) conformément à une force externe (F) exercée sur la première structure (11, 17; 21; 31) par un utilisateur,
- générer un signal de codeur (S2) au moyen d'un codeur rotatif incrémental (50) couplé à l'arbre de rotor (41) du moteur pas à pas (40) et ce conformément à la modification de la position angulaire de l'arbre de rotor (41),
- déterminer, sur la base du signal de codeur (S2), un signal de déviation positionnelle (S4) caractérisant une déviation de la position angulaire (S3) de l'arbre de rotor (41) par rapport à une position angulaire de référence (S1) de l'arbre de rotor (41), et
- commander le moteur pas à pas (40) de façon à ce qu'il fasse tourner l'arbre de rotor (41) en fonction du signal de déviation positionnelle (S4) de façon à ce que le moteur pas à pas (40) supporte un déplacement de la première structure (11, 17; 21; 31) qui est relatif à la deuxième structure (14, 15; 22; 32) et conforme à la force externe exercée (F).
